# EUROPÄISCHE PATENTSCHRIFT

(11) **EP 3 528 688 B1**
(45) Veröffentlichungstag und Bekanntmachung des Hinweises auf die Patenterteilung: **28.07.2021**
(21) Anmeldenummer: 18765554.3
(22) Anmeldetag: 23.08.2018
(51) Int. Cl.: A61B 1/00, A61B 1/005, A61B 1/012, A61B 17/16

(54) **MEDIZINISCHE VORRICHTUNG**
MEDICAL DEVICE
DISPOSITIF MÉDICAL

(30) Priorität: 27.10.2017 DE 102017010033
(43) Veröffentlichungstag der Anmeldung: 28.08.2019
(73) Patentinhaber: Joimax GmbH, 76227 Karlsruhe (DE)
(72) Erfinder: RIES, Wolfgang, 76351 Linkenheim (DE); SCHENDZIELORZ, Lars, 76351 Linkenheim (DE); STEEGMÜLLER, Rainer, 70839 Gerlingen (DE)
(74) Vertreter: Lichti - Patentanwälte Partnerschaft mbB
(86) Internationale Anmeldenummer: PCT/EP2018/000412
(87) Internationale Veröffentlichungsnummer: WO 2019/081051

(56) Entgegenhaltungen:
- EP-A1- 2 790 596
- DE-A1- 10 156 917
- DE-A1-102015 204 946
- US-A- 5 938 616
- US-A1- 2013 165 908
- US-A1- 2015 231 365
- US-A1- 2016 015 251

## Beschreibung

Die Erfindung betrifft eine medizinische Vorrichtung mit einem Schwenkkopf.

Eine derartige medizinische Vorrichtung ist grundsätzlich aus der EP 2 790 596 B1 bekannt. Bei dieser Vorrichtung ist zur nichtaxialen Ausrichtung des Arbeitskopfes eines drehbaren chirurgischen Werkzeugs, wie eines Fräsers oder eines Bohrers bei einem Führungsteil der Vorrichtung am distalen Ende ein relativ zur Längsachse der Vorrichtung seitlich abgebogener Führungsabschnitt vorgesehen.

Weiter ist aus der DE 100 36 108 A1 ein chirurgisches Instrument bekannt, das an seinem distalen Ende eine gehäuseartige Aufnahme zur drehbaren Lagerung eines Maulteils hat, die an einem distalen Ende eines Hohlschaftes verschwenkbar angelenkt ist. Die Betätigung erfolgt vom proximalen Ende über eine Betätigungsstange mittels eines an dieser proximal angreifenden Getriebemechanismus von einer an einem proximalen Hebel angreifenden Hand. Der Bedienungsmechanismus ist äußerst aufwendig ausgebildet, weist einen erheblichen seitlichen oder radialen Platzbedarf auf und erfordert Bedienung mit der gesamten Hand, einschließlich Bewegung derselben relativ zum Arm eines Bedieners.

Die DE 101 56 917 A1 zeigt ein Instrument für die endoskopische Chirurgie mit einem Gehäuse, mit einem mit seinem proximalen Ende an dem Gehäuse arretierbaren rohrförmigen Schaft, mit einem am distalen Ende des Schafts angeordneten Werkzeug und mit einem durch den Schaft hindurchtretenden und in diesem längsverschiebbaren Betätigungsorgan für das Werkzeug, wobei das Betätigungsorgan mit seinem proximalen Ende in eine im Wesentlichen zylindrische Gehäuseöffnung eingreift und dort mit einem schwenkbar am Gehäuse gelagerten Griffteil über einen im Gehäuse angeordneten Kupplungsmechanismus derart koppelbar ist, dass ein Verschwenken des Griffteils eine Längsverschiebung des Betätigungsorgans und eine Betätigung des Werkzeugs bewirkt. Um das Werkzeug zusammen mit dem Betätigungsorgan auf einfache Weise aus dem Gehäuse entfernen zu können, ist vorgesehen, dass der Kupplungsmechanismus ein in der zylindrischen Gehäuseöffnung längsverschiebbar angeordnetes, ein zu dieser koaxiale Aufnahmeöffnung für das proximale Ende des Betätigungsorgans aufweisendes Verschiebestück umfasst.

Die US 2016/0015251 A1 zeigt eine Betätigungsvorrichtung, die ein flexibles längliches Element für medizinische Zwecke eine vorbestimmte Aktion ausführen lässt, mit einem Druck-/Zugelement und einem Betätigungselement. Das Druck-/Zugelement umfasst zwei bewegliche Abschnitte, die auf einer proximalen Seite angeordnet sind und relativ zueinander beweglich sind, sowie zwei sich vom jeweiligen beweglichen Abschnitt erstreckende Abschnitte. Das Druck-/Zugelement wird in Verbindung mit einer Bewegung der beweglichen Abschnitte gedrückt/gezogen. Das Betätigungselement ist in Umfangsrichtung eines länglichen Elements drehbar. Das Betätigungselement umfasst zwei Führungsabschnitte, die jeweils die beiden beweglichen Abschnitte führen. Das Druck-/Zugelement kann das längliche Element dazu bringen, eine Vorwärts-/Rückzugsaktion und/oder eine Biegebewegung auszuführen.

Der Erfindung liegt daher die Aufgabe zugrunde, eine Vorrichtung mit Schwenkkopf dahingehend weiterzubilden, das bei einfacher Ausgestaltung, geringem Platzbedarf für die Ausbildung des Schwenkmechanismus an sich unter Freihaltung eines Hohlraumes für ein chirurgisches Werkstück einerseits und andererseits einfacher und platzsparender Ausbildung der proximalen Bedienungselemente eine exakte Schwenkpositionierung eines distalen Schwenkelementes und damit der Ausrichtung eines durch dieses bestimmten Werkzeugkopfes gegeben ist.

Erfindungsgemäß wird die genannte Aufgabe bei einer medizinischen Vorrichtung mit Schwenkkopf durch die Merkmale des Anspruchs 1 gelöst.

In bevorzugter Ausgestaltung ist vorgesehen, dass die Axialbewegung des Führungsrohrs über eine Kulissenführung mit einem unter einem Winkel ungleich ≠ 90° zur Achse sich in Umfangsrichtung erstreckenden Schlitz sowie einen in diesem geführten Stift erfolgt, wobei weiterhin der Stift fest mit dem Führungsrohr verbunden ist und der Schlitz an einem mit dem Bedienungselement fest, insbesondere einstückig ausgebildeten Zylindermantel ausgebildet ist.

Darüber hinaus sehen bevorzugte Weiterbildungen vor, dass die Stifte jeweils ein radial gerichtetes, sich in Richtung der Achse erstreckendes Langloch in einem mit dem Führungsrohr fest verbundenen Teil, insbesondere einem Zylinderteil durchgreifen. Um insbesondere im distalen Bereich den nötigen Freiraum zum Durchtritt der Antriebswelle eines Werkzeugs zu gewährleisten sehen Weiterbildungen vor, dass das Betätigungsrohr mit einer Lasche exzentrisch in einem proximalen Bereich des Schwenkkopfs zum Verschwenken desselben angreift, wobei in Umfangsrichtung unter einem Winkel ungleich 90° zur Achse aufeinanderfolgende Rastvertiefungen in einem mit dem Führungsrohr verbundenen Zylindermantelteil und einen in diese eingreifenden mit dem Bedienungselement verbundenen Federstift.

Eine genaue Positionierung der Winkelstellung kann durch eine relativ zur Führungseinheit drehbare Dreheinheit erreicht werden.

Darüber hinaus kann die erfindungsgemäße Vorrichtung in bevorzugter Ausgestaltung zur Verbindung mit der Abtriebswelle eines Drehantriebs dahingehend ausgestaltet sein, dass die Dreheinheit ein zweites Kopplungsteil aufweist, das im ersten Kopplungsteil axialfest, aber drehbar angeordnet ist, wobei vorzugsweise die Dreheinheit, insbesondere deren zweites Kopplungsteil Kopplungsschlitze zur drehfesten Verbindung mit einer Abtriebswelle des Druckantriebs aufweist
(weiter auf Seite 4 der ursprünglichen Unterlagen) und das erste Kopplungsteil Formausbildungen - vorzugsweise in Form von Schlitzen und einer Ringnut - zur axial- und drehfesten Verbindung mit einem Antrieb und/oder dem Gehäuse eines Antriebs aufweist.

Weiterbildungen sehen dabei ein relativ zur Führungs- und Betätigungseinheit drehbares aber zu diesem axial festlegbares chirurgisches Drehwerkzeug vor, wobei insbesondere das Drehwerkzeug am proximalen Ende eines Schaftes eine nicht zylindrische, vorzugsweise Vierkantausbildung zum drehfesten Eingriff in eine entsprechende nicht zylindrische, vorzugsweise Vierkantausnehmung der zweiten Kopplungseinheit aufweist.

Darüber hinaus kann vorzugsweise vorgesehen sein, dass ein Schaft des Werkzeugs mit einem verjüngten Bereich in den Durchbruch eines Freigabe- und Blockierelements des ersten Kopplungsteils hindurchgreift.

Weitere bevorzugte Ausgestaltungen zeichnen sich dadurch aus, dass das Betätigungsrohr koaxial im Führungsrohr angeordnet ist und/oder das der Außendurchmesser des Betätigungsrohrs dem Innendurchmesser des Führungsrohrs entspricht.

Die Kopplung der Kopplungselemente kann in bevorzugter Weise derart erfolgen wie dies in der EP 2 393 435 gezeigt ist. Die vorstehend angegebenen Verbindungsausbildungen der Kopplungselemente sind derart ausgestaltet, dass eine Verbindung in jener Druckschrift beschriebenen mit einem Antrieb möglich ist. Insofern wird diesbezüglich der Offenbarungsgehalt der genannten Druckschrift vollständig zum Offenbarungsgehalt der vorstehenden Anmeldung gemacht.

Weitere Vorteile und Merkmale der Erfindung ergeben sich aus den Ansprüchen aus der nachfolgenden Beschreibung, in der ein Ausführungsbeispiel der Erfindung unter Bezugnahme auf die Zeichnung im Einzelnen erläutert ist. Dabei zeigt:
- Fig. 1: eine Seitenansicht der erfindungsgemäßen medizinischen Vorrichtung;
- Fig. 2: eine Seitenansicht unter einem anderen Angularwinkel, teilweise geschnitten;
- Fig. 3: die Seitendarstellung der Figur mit einem anderen Abstand in einen äußeren Teil zur Darstellung der Ausbildung eines unterhalb liegenden Teils;
- Fig. 4: einen Längsschnitt durch die erfindungsgemäße Vorrichtung entsprechend AB der Fig. 2;
- Fig. 5: einen Längsschnitt unter einem Angularwinkel um 90° zum Schnitt der Fig. 4; und
- Fig. 6: eine vergrößerte Darstellung des Schwenkkopfs der Fig. 2.

Die erfindungsgemäße Vorrichtung 1 weist zunächst ein Führungsteil 2 auf. Das Führungsteil 2 ist mit einem Motorantrieb bzw. dem Gehäuse eines Motorantriebs entsprechend der EP 2 393 435 fest, d.h. dreh- und axialfest, verbunden. Die Festlegung geschieht über die proximalen Längsschlitze 2.1a und die Ringnut 2.1b eines Kopplungsteils 2.1 des Führungsteils 2 in der dort beschriebenen Weise.

Mit dem Kopplungsteil 2.1 ist fest, d.h. dreh- und axialfest sowie unlösbar ein Führungsrohr 2.2 des Führungsteils 2 verbunden. Am distalen Ende des Führungsrohrs 2.2 ist an diesem schwenkbar ein zylindermantelförmiger Schwenkkopf 2.3 angeordnet. Die gelenkige Verbindung wird durch auf der Höhe einer Mittelachse A des Führungsrohrs 2.2 und des ersten Kopplungsteils 2.1 diametral einander gegenüberliegenden Gelenke 2.3.1 gebildet.

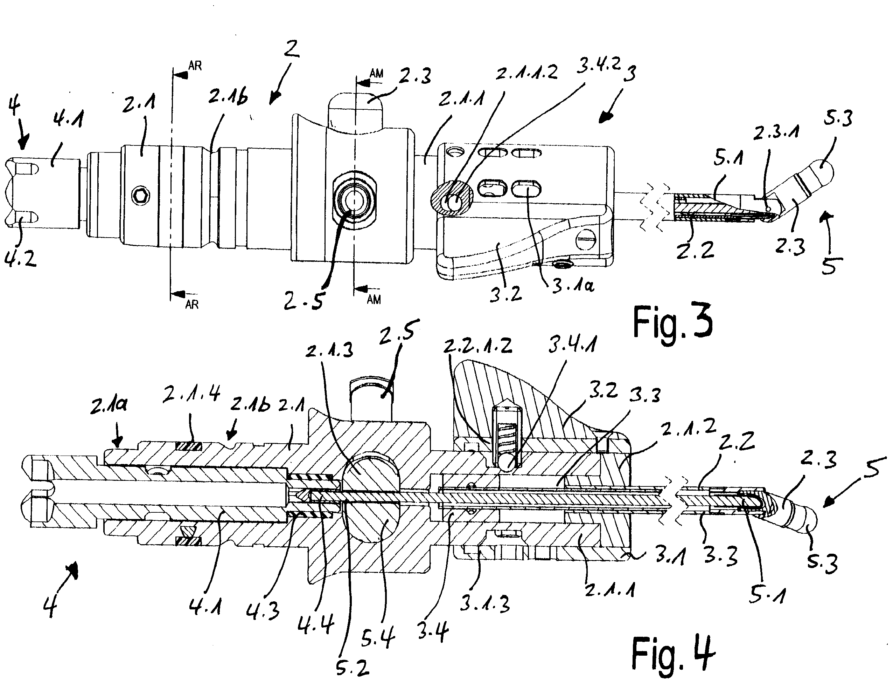

Einstückig mit dem ersten Kopplungsteil 2.1 ist distal zu diesem ein erster Zylindermantel 2.1.1 ausgebildet (auch Fig. 4), in welches am distalen Ende ein Rohrverbindungsteil 2.1.2 (Fig. 4) eingesetzt ist, mit dem das Führungsrohr 2.2 verbunden ist und zwar vorzugsweise stoffschlüssig. Die genannten Teile 2.1, 2.1.1, 2.1.2 und 2.2 bestehen vorzugsweise aus Edelstahl, wobei die stoffschlüssigen Verbindungen zwischen einerseits dem Zylindermantel 2.1.1 und dem Rohrverbindungsteil 2.1.2 in Form einer Buchse und andererseits zwischen letzterem und dem Führungsrohr 2.2 jeweils durch Laserschweißung gebildet sind.

Etwa mittig des Kopplungsteils 2.1 ist ein durch eine Feder (nicht dargestellt) vorgespanntes Freigabe- und Blockierelement 2.1.3 in Form eines Druckknopfs erkennbar, dessen Funktion weiter unten beschrieben wird.

Relativ zum Führungsteil 2 ist eine Betätigungseinheit 3 beschränkt drehbar und axial beweglich vorgesehen.

Die Betätigungseinheit 3 weist hierzu einen den ersten Zylindermantel 2.1.1 beweglich umgebenden zweiten Zylindermantel 3.1 mit einem in Umfangsrichtung der Achse A schwenkbeweglichen Betätigungselement 3.2 in Form einer Lasche auf, die beide einstückig miteinander ausgebildet sind. Der zweite Zylindermantel 3.1 ist einseitig des Betätigungselements 3.2 mit einem Schlitz 3.1.1 versehen, der sich in Umfangsrichtung des zweiten Zylindermantels 3.1 erstreckt und zwar unter einem Winkel zur Achse A ≠ 90°, also nicht senkrecht zur Achse A ausgerichtet ist. Der Erstreckungswinkel des Schlitzes 3.1.1 zur Senkrechten zur Achse A beträgt nur wenige Grad, vorzugsweise 3° bis 5°. In den Schlitz 3.1.1 ragt ein fest mit dem ersten Zylindermantel 2.1.1 verbundener Stift 2.4, beispielsweise in Form einer eingeschraubten kopflosen Schlitzschraube. Der Schlitz 3.1.1 bildet damit eine Kulissenführung für den Stift 2.4. Am Führungsteil 2 ist seitlich ein Luer-Lock 2.5 vorgesehen, um ein Spülen zu ermöglichen.

Kurze Axialschlitze 3.1a im Zylindermantel 3.1 der Betätigungseinheit erlauben eine bessere Reinigungsmöglichkeit durch Durchspülen und tragen zur Gewichtsersparnis bei.

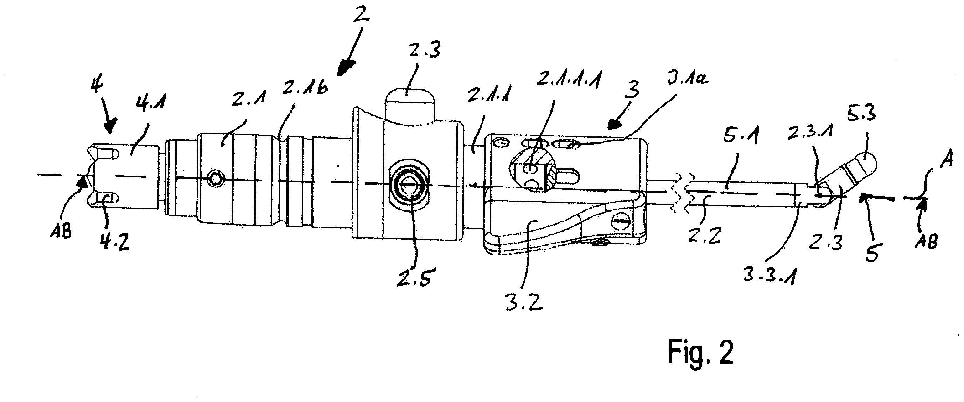

Wie in Fig. 2 ersichtlich ist, sind proximal der Anordnung der Stifte 2.4 in der Außenwandung des Zylindermantels 2.1.1 in Umfangsrichtung, ebenfalls unter dem gleichen Winkel wie der Schlitz 3.1.1 zur Achse A ausgerichtet, mehrere Vertiefungen 2.1.1.1 ausgebildet, in die - wie in Fig. 4 ersichtlich ist - eine durch eine Feder 3.1.2.1 vorgespannte Kugel 3.1.2.2 eines im Zylindermantel 3.1 gelagerten Federstifts 3.1.2 jeweils in durch die Position der Vertiefungen 2.1.1.1 bestimmte Winkelstellungen einrasten kann.

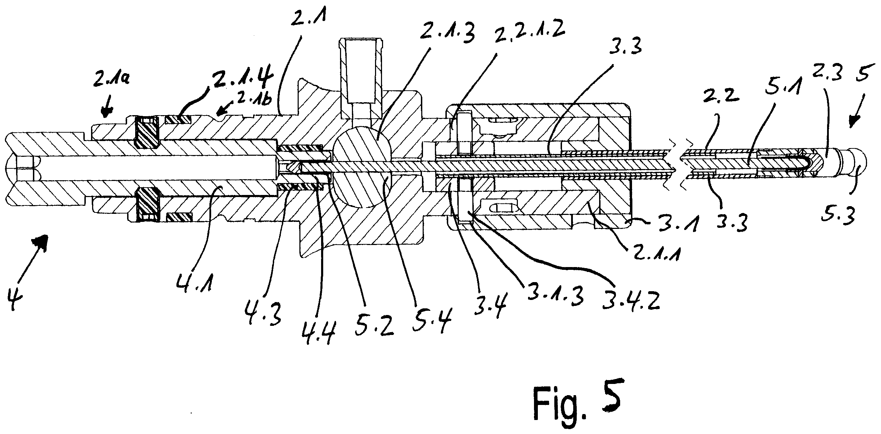

Wie in den Fig. 3 und 5 dargestellt ist, erstreckt sich axial verschiebbar ein Betätigungsrohr 3.3 für den Schwenckopf 2.3. Das Betätigungsrohr 3.3 ist proximal fest, d.h. dreh- und axialfest mit einer Buchse 3.4 (Fig. 4) verbunden, vorzugsweise stoffschlüssig, insbesondere durch Laserschweißung. Die Buchse 3.4 weist zwei einander diametral gegenüberliegende radiale Gewindebohrungen 3.4.1 (Fig. 5) auf, in die radial Stifte 3.4.2 in Form von kopflosen Schrauben eingeschraubt sind. Die Stifte 3.4.2 erstrecken sich durch Radialdurchbrüche 2.1.1.2, die in diesem Bereich im ersten Zylindermantel 2.1.1 in Axialrichtung als Langlöcher ausgebildet sind, so dass die Stifte 3.4.2 über eine begrenzte Strecke in diesen Radialdurchbrüchen 2.1.1.2, axial verschiebbar oder beweglich sind und mit ihnen das über die Buchse 3.4 verbundene Betätigungsrohr 3.3 (Fig. 5). Radial außenliegende Enden der Stifte 3.4.2 ragen in eine den Querabmessungen der Stifte entsprechende radial nach innen gerichtete Vertiefung 3.1.3 des zweiten Zylindermantels 3.1 der Betätigungseinheit 3.

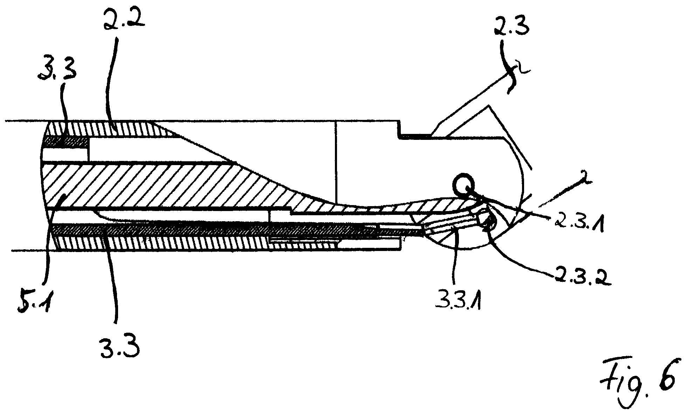

Wie in der Fig. 6 dargestellt ist, setzt sich das Betätigungsrohr 3.3 an seinem distalen Ende in einer distalen Lasche 3.3.1 fort, die beidseitig exzentrisch zu den Gelenken 2.3.1 am Schwenkkopf 2.3 angeordnete Stifte 2.3.2 umgreift.

Die Längsschnitte der Fig. 4, 5 zeigen am Außenumfang des Kopplungsteils 2.1 Magnete 2.1.4 zur Erkennung der mit dem Antrieb (nicht dargestellt) verbundenen Vorrichtung, damit wird diese Drehzahl und Drehrichtung automatisch angepasst.

Die Betätigung des Schwenkkopfes 2.3 geschieht aufgrund der beschriebenen Ausgestaltung folgendermaßen:
Wird der zweite Zylindermantel 3.1 durch Angreifen eines Benutzers an der Lasche 3.2 und Verschwenken derselben um den ersten Zylindermantel 2.1.1 und damit relativ zum Kopplungsteil 2.1 verschwenkt, so wird der zweite Zylindermantel 3.1 gleichzeitig aufgrund der durch den Schlitz 3.1.1 und den Stift 2.4 gebildeten Kulissenführung axial in Richtung der Achse A bewegt. Diese Axialbewegung überträgt sich auf die Stifte 3.4.2 und über diese und die Buchsen 3.4 auf das Betätigungsrohr 3.3, das so ebenfalls axial bewegt wird. Diese Axialbewegung wird über die Lasche oder den Ansatz 3.3.1 und die exzentrisch angeordneten Stifte 2.3.2 auf den Schwenkkopf 2.3 übertragen und verschwenken diesen um seine durch die Gelenke 2.3.1 gebildete Schwenkachse aus einer gestreckten Position in eine zur Achse A in eine abgewinkelte Position, wie dies insbesondere in der Fig. 2 sichtbar ist.

Damit wird erreicht, dass ein sich durch das Führungsrohr 2.2 und Betätigungsrohr 3.3 hindurch erstreckendes chirurgisches Drehwerkzeug mit einem distalen Arbeitskopf in einer Winkelstellung zur Achse, insbesondere der in Fig. 2 dargestellten Winkelstellung arbeiten kann.

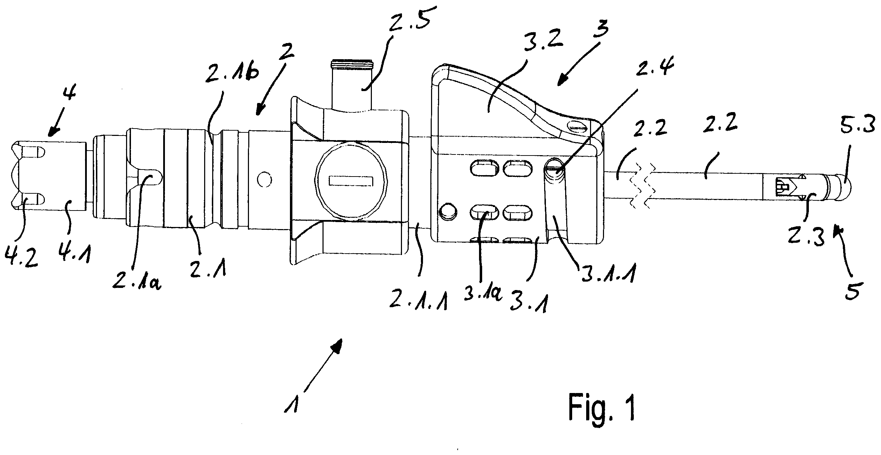

In den Figuren 1 bis 5 ist weiterhin - links - eine Rotationseinheit 4 dargestellt. Diese weist zunächst ein im ersten Kopplungsteil 2.1 drehbar gelagertes zweites Kopplungsteil 4.1 auf, das an seinem proximalen Ende Kopplungselemente in Form von zur Achse A koaxialen Schlitzen aufweist und derart mit der Abtriebsachse eines Drehantriebs koppelbar ist, wie dies in der EP 2 393 435 offenbart ist.

Das zweite Kopplungsteil 4.1 ist im ersten Kopplungsteil 2.1 zwar drehbar, insbesondere über eine Lagerung 4.3, aber axialfest gelagert (Fig. 3, 5). Es weist distal eine sich axial erstreckende im Querschnitt nicht kreissymmetrische Ausnehmung 4.4, beispielsweise eine Vierkantaufnehmung auf, in die das proximale Ende eines Schafts 5.1, wie beispielweise eines Bohrers oder Fräsers vom distalen Ende der Vorrichtung her, also vom Schwenkkopf 2.3 her drehfest eingesteckt und eingesetzt werden kann. Das Drehwerkzeug 5 weist hierzu zwar einen über den größten Teil seiner Längserstreckung zylindrischen Schaft 5.1 auf, dieser ist aber am proximalen Ende entsprechend der Ausnehmung 4.4 als Mehrkant 5.2, insbesondere Vierkant, ausgebildet. Am distalen Ende des Werkzeugs 5 ist ein Werkzeugkopf 5.3, beispielsweise in Form eines Fräs- oder Bohrkopfes vorgesehen.

Die axiale Festlegung des Werkzeugs 5 erfolgt über den schon erwähnten Druckknopf 2.1.3, der einen Axialdurchbruch aufweist, durch den bei Drücken des Druckknopfes 2.1.3 zum Einstecken und Entnehmen des Werkzeugs 5 der Schaft 5.1 einschließlich des Vierkants 5.2 an seinem proximalen Ende bis in die Vertiefung 4.4 hindurchsteckbar ist und bei Entlasten des Druckknopfs 2.1.3 unter der Wirkung der genannten nicht dargestellten Federn den Schaft 5.1 über einen verjüngten Bereich 5.4 axial festlegt.

## Patentansprüche

1. Medizinische Vorrichtung mit einer Führungseinheit (2), die ein Führungsrohr (2.2) mit einer Längsachse (A) und ein fest mit diesem verbundenes proximales erstes Kopplungsteil (2.1) aufweist, wobei die Führungseinheit distal einen zylindermantelförmigen über diagonal gegenüberliegende am distalen Ende des Führungsrohrs (2.2) ausgebildete Gelenke (2.3.1) relativ zum Führungsrohr (2) verschwenkbaren Schwenkkopf (2.3) aufweist, wobei weiter im Führungsrohr (2.2) ein mit dem Schwenkkopf (2.3) verbundenes Betätigungsrohr (3.3) axial beweglich ist, das durch ein proximales Bedienungselement (3.2) ein Verschwenken des Schwenkkopfes bewirkt und dass das Bedienungselement (3.2) um die Längsachse (A) verschwenkbar ist und unter Axialverschiebung des Betätigungsrohrs (3.3) das Verschwenken des Schwenkkopfes (2.3) bewirkt.

2. Vorrichtung nach Anspruch 1, **dadurch gekennzeichnet, dass** die Axialbewegung des Führungsrohrs (2.2) über eine Kulissenführung mit einem unter einem Winkel ungleich ≠ 90° zur Achse (A) sich in Umfangsrichtung erstreckenden Schlitz (3.1.1) sowie einen in diesem geführten Stift (2.4) erfolgt.

3. Vorrichtung nach Anspruch 2, **dadurch gekennzeichnet, dass** der Stift (2.4) fest mit dem Führungsrohr (2.2) verbunden ist und der Schlitz (3.1.1) an einem mit dem Bedienungselement (3.2) fest, insbesondere einstückig ausgebildeten Zylindermantel (3.1) ausgebildet ist.

4. Vorrichtung nach einem der vorangehenden Ansprüche, **dadurch gekennzeichnet, dass** mit dem Führungsrohr (3.3) sich radial erstreckende Stifte (3.4.2) verbunden sind, die jeweils in eine nach innen gerichtete Führungsnut mit ihrem Durchmesser entsprechender Breite in axialer Richtung des Bedienungselementes (3.2) eingreifen.

5. Vorrichtung nach Anspruch 4, **dadurch gekennzeichnet, dass** die Stifte (3.4.2) jeweils ein radial gerichtetes, sich in Richtung der Achse (A) erstreckendes Langloch in einem mit dem Führungsrohr (2.2) fest verbundenen Teil, insbesondere einem Zylinderteil (2.1.1) durchgreifen.

6. Vorrichtung nach einem der vorangehenden Ansprüche, **dadurch gekennzeichnet, dass** das Betätigungsrohr (3.3) mit einer Lasche (3.2) exzentrisch in einem proximalen Bereich des Schwenkkopfs (2.3) zum Verschwenken desselben angreift.

7. Vorrichtung nach einem der vorangehenden Ansprüche, **gekennzeichnet durch** in Umfangsrichtung unter einem Winkel ungleich 90° zur Achse (A) aufeinanderfolgende Rastvertiefungen (2.2.1.1) in einem mit dem Führungsrohr (2.2) verbundenen Zylindermantelteil (2.1.1) und einen in diese eingreifenden mit dem Bedienungselement (3.2) verbundenen Federstift (3.1.1).

8. Vorrichtung nach einem der vorangehenden Ansprüche, **gekennzeichnet durch** eine relativ zur Führungseinheit (2) drehbare Dreheinheit (4).

9. Vorrichtung nach Anspruch 8, **dadurch gekennzeichnet, dass** die Dreheinheit (4) ein zweites Kopplungsteil (4.1) aufweist, das im ersten Kopplungsteil (2.1) axial fest, aber drehbar angeordnet ist.

10. Vorrichtung nach Anspruch 8 oder 9, **dadurch gekennzeichnet, dass** die Dreheinheit (4), insbesondere das zweite Kopplungsteil (4.1) Kopplungsschlitze (4.2) zur drehfesten Verbindung mit einer Antriebswelle aufweist.

11. Vorrichtung nach einem der vorangehenden Ansprüche, **dadurch gekennzeichnet, dass** das erste Kopplungsteil (2.1) Formausbildungen (2.1a, 2.1b) zur axial- und drehfesten Verbindung mit einem Antrieb und/oder dem Gehäuse eines Antriebs aufweist.

12. Vorrichtung nach einem der vorangehenden Ansprüche, **gekennzeichnet durch** ein relativ zur Führungs- und Betätigungseinheit (2, 3) drehbares aber zu diesem axial festlegbares chirurgisches Drehwerkzeug (5).

13. Vorrichtung nach Anspruch 12, **dadurch gekennzeichnet, dass** das Drehwerkzeug (5) am proximalen Ende eines Schaftes (5.1) eine nicht zylindrische, vorzugsweise Vierkantausbildung (4.4) zum drehfesten Eingriff in eine entsprechende nicht zylindrische, vorzugsweise Vierkantausnehmung der zweiten Kopplungseinheit (4.1) aufweist.

14. Vorrichtung nach Anspruch 12 oder 13, **dadurch gekennzeichnet, dass** ein Schaft (5.1) des Werkzeugs (5) mit einem verjüngten Bereich in den Durchbruch eines Freigabe- und Blockierelements (2.1.3) des ersten Kopplungsteil (2.1) hindurchgreift.

15. Vorrichtung nach einem der vorangehenden Ansprüche, **dadurch gekennzeichnet, dass** das Betätigungsrohr (3.3) koaxial im Führungsrohr (2.2) angeordnet ist.

16. Vorrichtung nach einem der vorangehenden Ansprüche, **dadurch gekennzeichnet, dass** der Außendurchmesser des Betätigungsrohrs (3.3) dem Innendurchmesser des Führungsrohrs (2) entspricht.

## Claims

1. Medical device comprising a guide unit (2) having a guide tube (2.2) with a longitudinal axis (A) and a proximal first coupling part (2.1) being fixedly connected to the guide tube (2.2), wherein the guide unit comprises a distal pivoting head (2.3) with a cylindrical surface, wherein the pivoting head (2.3) is pivotable relative to the guide tube (2) via diagonally opposite joints (2.3.1) formed at the distal end of the guide tube (2.2), wherein said medical device also comprising an actuating tube (3.3) which is axially movable in the guide tube (2.2) and is connected to the pivoting head (2.3), causing a pivoting movement of the pivoting head (2.3) by means of a proximal operating element (3.2) wherein the operating element (3.2) is pivotable about the longitudinal axis (A) and causes the pivoting movement of the pivoting head (2.3) with axial displacement of the actuating tube (3.3).

2. Device according to claim 1, **characterized in that** the axial movement of the guide tube (2.2) is carried out via a link guide having a slot (3.1.1) which extends in the circumferential direction at an angle not equal to ≠ 90° to the axis (A) and a pin (2.4) guided therein.

3. Device according to claim 2, **characterized in that** the pin (2.4) is fixedly connected to the guide tube (2.2) and the slot (3.1.1) is formed on a cylinder jacket (3.1) which is fixed, in particular in one piece, to the operating element (3.2).

4. Device according to any of the preceding claims, **characterized in that** radially extending pins (3.4.2) are connected to the guide tube (3.3), each engaging into an inwardly oriented guide groove having a diameter corresponding to the width thereof in the axial direction of the operating element (3.2).

5. Device according to claim 4, **characterized in that** the pins (3.4.2) each engage through a radially oriented elongated hole extending in the direction of the axis (A) in a part which is fixedly connected to the guide tube (2.2), in particular a cylinder part (2.1.1).

6. Device according to any of the preceding claims, **characterized in that** the actuating tube (3.3) acts eccentrically with a tab in a proximal area of the pivoting head (2.3) for the pivoting movement thereof.

7. Device according to any of the preceding claims, **characterized by** latching depressions (2.2.1.1) which are arranged next to one another in the circumferential direction at an angle unequal to 90° to the axis (A) in a cylinder jacket part (2.1.1) connected to the guide tube (2.2) and a spring pin (3.1.1) connected to the operating element (3.2) and engaging in the latter.

8. Device according to any of the preceding claims, **characterized by** a rotating unit (4) which is rotatable relatively to the guide unit (2).

9. Device according to claim 8, **characterized in that** the rotating unit (4) has a second coupling part (4.1) which is arranged axially fixed but rotatable in the first coupling part (2.1).

10. Device according to claim 8 or 9, **characterized in that** the rotating unit (4), in particular the second coupling part (4.1), has coupling slots (4.2) for the rotationally fixed connection to a drive shaft.

11. Device according to any of the preceding claims, **characterized in that** the first coupling part (2.1) has formations (2.1a, 2.1b) for axially and rotationally fixed connection to a drive and/or the housing of a drive.

12. Device according to any of the preceding claims, **characterized by** a surgical rotating tool (5) which is rotatable relative to the guide and actuating unit (2, 3) but can be axially fixed to the latter.

13. Device according to claim 12, **characterized in that** the rotating tool (5) has, at the proximal end of a shank (5.1), a non-cylindrical, preferably square design (4.4) for non-rotatable engagement in a corresponding non-cylindrical, preferably square recess of the second coupling unit (4.1).

14. Device according to claim 12 or 13, **characterized in that** a shank (5.1) of the tool (5) having a tapered area engages in the opening of a release and blocking element (2.1.3) of the first coupling part (2.1).

15. Device according to any of the preceding claims, **characterized in that** the actuating tube (3.3) is arranged coaxially in the guide tube (2.2).

16. Device according to any of the preceding claims, **characterized in that** the outer diameter of the actuating tube (3.3) corresponds to the inner diameter of the guide tube (2).

## Revendications

1. Dispositif médical équipé d'une unité de guidage (2) comportant un tube de guidage (2.2) avec un axe longitudinal (A) et une première partie de couplage (2.1) proximale reliée fixement à elle, l'unité de guidage comportant distalement une tête pivotante (2.3) en forme d'enveloppe de cylindre pouvant pivoter par rapport au tube de guidage (2) via des articulations (2.3.1) opposées en diagonale et réalisées au niveau de l'extrémité distale du tube de guidage (2.2), un tube d'actionnement (3.3) relié à la tête pivotante (2.3) pouvant en outre être mobile dans le plan axial dans le tube de guidage (2.2), ledit tube entraînant un pivotement de la tête pivotante par le biais de l'élément de commande (3.2) proximal et l'élément de commande (3.2) pouvant être pivoté autour de l'axe longitudinal (A) et entraînant le pivotement de la tête pivotante (2.3) par coulissement axial du tube d'actionnement (3.3).

2. Dispositif selon la revendication 1, **caractérisé en ce que** le mouvement axial du tube de guidage (2.2) se produit via un guidage à coulisse, avec une fente (3.1.1) s'étendant dans la direction périphérique selon un angle ≠ 90° par rapport à l'axe (A) ainsi que via une tige (2.4) guidée dans ledit tube.

3. Dispositif selon la revendication 2, **caractérisé en ce que** la tige (2.4) est reliée fixement au tube de guidage (2.2) et que la fente (3.1.1) est réalisée au niveau d'une enveloppe de cylindre (3.1) notamment d'un seul tenant, fixement avec l'élément de commande (3.2).

4. Dispositif selon l'une quelconque des revendications précédentes, **caractérisé en ce que** les tiges (3.4.2) s'étendant dans le plan radial sont reliées au tube de guidage (3.3), lesdites tiges s'engrenant respectivement dans une rainure de guidage orientée vers l'intérieur avec sa largeur correspondant au diamètre, dans la direction axiale de l'élément de commande (3.2).

5. Dispositif selon la revendication 4, **caractérisé en ce que** les tiges (3.4.2) agrippent respectivement un trou oblong orienté dans le plan radial et s'étendant dans la direction de l'axe (A) dans une partie reliée fixement au tube de guidage (2.2), notamment une partie de cylindre (2.1.1).

6. Dispositif selon l'une quelconque des revendications précédentes, **caractérisé en ce que** le tube d'actionnement (3.3) doté d'une bride s'engrène de façon excentrée dans une zone proximale de la tête pivotante (2.3) pour le pivotement de celle-ci.

7. Dispositif selon l'une quelconque des revendications précédentes, **caractérisé par** des renfoncements d'arrêt (2.2.1.1) successifs dans la direction périphérique selon un angle différent de 90° par rapport à l'axe (A) dans une partie d'enveloppe de cylindre (2.1.1) reliée au tube de guidage (2.2) et par une tige de ressort (3.1.1) s'engrenant dans celle-ci et reliée à l'élément de commande (3.2).

8. Dispositif selon l'une quelconque des revendications précédentes, **caractérisé par** la présence d'une unité pivotante (4) pouvant tourner par rapport à l'unité de guidage (2).

9. Dispositif selon la revendication 8, **caractérisé en ce que** l'unité pivotante (4) comporte une deuxième partie de couplage (4.1) disposée fixement dans le plan axial mais de façon à pouvoir pivoter dans la première partie de couplage (2.1) .

10. Dispositif selon la revendication 8 ou 9, **caractérisé en ce que** l'unité pivotante (4), notamment la deuxième partie de couplage (4.1), comporte des fentes de couplage (4.2) pour la liaison solidaire en rotation avec un arbre d'entraînement.

11. Dispositif selon l'une quelconque des revendications précédentes, **caractérisé en ce que** la première partie de couplage (2.1) comporte des formes (2.1a, 2.1b) pour la liaison solidaire dans le plan axial et en rotation avec un entraînement et/ou le carter d'un entraînement.

12. Dispositif selon l'une quelconque des revendications précédentes, **caractérisé par** un outil chirurgical tournant (5) pouvant pivoter par rapport à l'unité de guidage et d'actionnement (2, 3) mais pouvant être fixé à elle dans le plan axial.

13. Dispositif selon la revendication 12, **caractérisé en ce que** l'outil tournant (5) comporte au niveau de l'extrémité proximale d'une tige (5.1) une formation (4.4) non cylindrique de préférence carrée pour la mise en prise fixe en rotation dans un évidement non cylindrique de préférence carré correspondant de la deuxième unité de couplage (4.1).

14. Dispositif selon la revendication 12 ou 13, **caractérisé en ce qu'**une tige (5.1) de l'outil (5) s'imbrique avec une zone rétrécie dans le passage d'un élément de libération et de blocage (2.1.3) de la première partie de couplage (2.1).

15. Dispositif selon l'une quelconque des revendications précédentes, **caractérisé en ce que** le tube d'actionnement (3.3) est disposé coaxialement dans le tube de guidage (2.2).

16. Dispositif selon l'une quelconque des revendications précédentes, **caractérisé en ce que** le diamètre extérieur du tube d'actionnement (3.3) correspond au diamètre intérieur du tube de guidage (2).
